# EUROPEAN PATENT APPLICATION

(11) **EP 1 679 084 A1**
(43) Date of publication of application: **12.07.2006**
(21) Application number: 04727102.8
(22) Date of filing: 13.04.2004
(51) Int. Cl.: A61K 49/04

(54) **X-RAY CONTRAST MEDIUM CONTAINING LIPOSOME AND METHOD FOR PREPARATION THEREOF**

(30) Priority: 21.10.2003 JP 2003360769; 12.11.2003 JP 2003382983; 21.11.2003 JP 2003392678; 21.11.2003 JP 2003392679; 23.03.2004 JP 2004085168
(71) Applicant: Konica Minolta Medical & Graphic, Inc., Tokyo 163-0512 (JP)
(72) Inventor: UEDA, Eiichi, Konica Minolta Medical & Graphic Inc, Hino-shi, Tokyo 191-8511 (JP); KAWAKATSU, S., Konica Minolta Medical & Graphic, Hino-shi, Tokyo 191-8511 (JP); NAKAJIMA, A., Konica Minolta Medical & Graphic Inc, Hino-shi, Tokyo 191-8511 (JP); NAGAIKE, Chiaki, Konica Minolta Medical & Graphic, Hino-shi, Tokyo 191-8511 (JP)
(74) Representative: Rees, Alexander Ellison
(86) International application number: PCT/JP2004/005261
(87) International publication number: WO 2005/037325

(57) **Abstract**

A radiographic contrast medium is disclosed, comprising liposomes having a narrow vesicle size distribution and comprising a lipid membrane containing a polyalkylene oxide group-containing compound and/or sterols, and the liposomes enclosing a water-soluble nonionic iodine compound. The liposomes which enclose a water phase inside the lipid membrane are dispersed in an aqueous medium, and preferably, both of the water phase and the aqueous medium contain the iodine compound and an additive and each of an iodine compound concentration and an additive concentration is substantially the same in both of the water phase and the aqueous medium, in which the weight ratio of the iodine compound to the lipid membrane is 1 to 10. The contrast medium is prepared by the supercritical carbon dioxide method and therefore, is substantially free of a toxic solvent such as a chlorine-containing solvent.

## Description

### FIELD OF THE INVENTION

The present invention relates to a non-oral contrast medium for use in radiography and in particular to a radiographic contrast medium which contains a liposome including a contrast medium material.

### BACKGROUND OF THE INVENTION

Simple X-ray photography and computer tomography are the nucleus of current medical image diagnosis. So-called hard tissues such as bones and teeth efficiently absorb X-rays and thereby high contrast X-ray images can be obtained. On the contrary, the difference in X-ray absorption between different soft tissues is relatively small, making it difficult to obtain high contrast images. In such cases, contrast mediums are generally used to obtain high contrast images.

Almost all X-ray contrast mediums which are currently of practical use are contrast medium materials which are water-solubilized compounds containing a triiodophenyl group. The contrast medium is given to a lumen region such as a vascular tract, a ureter or a uterine tube to be used for examination of a form or stenosis of the lumen. However, the foregoing compounds are promptly discharged from the lumen region without interacting with tissue or disease regions, which is not useful for detailed examination of the tissue or disease region, specifically such as cancer tissue. Therefore, an X-ray contrast medium has been desired which can be selectively accumulated in/or onto the targeted tissue or disease region, thereby giving an image which can be distinguished with clear contrast from the circumference or other regions. Such a contrast medium enables precise detection of even a minute amount of cancer tissue. Not only X-ray radiography but also other methods based on different measurement principles have been developed as an examination method to locate tumors. For example, MRI (magnetic resonance imaging) exhibits limited sensitivity to precisely image cancer tissue, specifically such as minute amounts cancer tissue and PET (positive emission tomography) is not popular in terms of exposure to radiation and operating cost, both of which require large-sized equipment and expensive apparatus and are still not a generally used examination method.

International Publication WO98/46275, ibid WO95/31181, ibid WO96/28414, ibid WO96/00089; U.S. Patent Nos. 4,873,075 and 4,567,034 disclose methods in which a hydrophobic iodine compound is dispersed in water in the presence of a surfactant or a fat to image a tumor, liver, spleen, adrenal cortex, arteriosclerosis plexus, vessel and lymphatic system. In the foregoing methods, the use of fine-grained contrast medium extends its internal residence time to selectively enhance the image contrast of the disease region. However, a pharmaceutical preparations proposed for that purpose were insufficient in terms of contrast-enhancing efficiency and selectivity. Moreover, an iodine compound used therein was hydrophobic so that external discharge after imaging is retarded, producing problems such as increased discomfort to the patient.

Further, a method in which a contrast medium compound was allowed to be included in a liposome which was comprised of a lipid similar to a biomembrane, and which exhibited high safety due to its low antigenicity, was studied as a technique for producing a contrast medium in the form of fine particles. International Publication WO88/09165, ibid WO89/00988, ibid WO90/07491; JP-A No. 07-316079 and 2003-5596 (hereinafter, the term JP-A refers to Japanese Patent Application Publication) propose a liposome containing an ionic or non-ionic contrast medium. Japanese Patent No. 2619037 discloses a method in which a water-soluble iodine compound is included in a liposome, which is intended to enhance organ selectivity by increasing the quantity of the iodine compound in the liposome to 1.5 to 6 g/g lipid. However, this method was proved to be low in selectivity for tumors. This was assumed to be due to the relatively large particles having sizes of 0.5 to 1.0 µm. Particles of ca. 0.1 µm are supposed to be preferable for accumulation onto tumor tissue, however, it was difficult in the method described above to obtain fine particles and decreasing the vesicle size resulted in a decreased quantity ratio of iodine compound to lipid. Further, in the above-mentioned methods, although a liposome exhibiting high safety as raw material and optimal degradability in vivo, organic solvents, specifically chlorine-containing solvents such as chloroform and dichloromethane were used in the preparation process, as a solvent for phospholipid forming a liposome membrane. Accordingly the foregoing methods were not practically applicable due to toxity of retained solvents.

On the other hand, although chemicals soluble in lipid are easily included in a liposome, the included quantity, depending on other factors, is not necessarily large. Although water-soluble electrolytic chemicals can be included in a liposome through interaction of a charge of the chemicals with that of a charged lipid, such a means is not applicable to water-soluble non-electrolytic chemicals. It has been generally desired to allow non-ionic iodine compounds substantially exhibiting low toxicity to be included in a liposome rather than ionic contrast medium compounds, which is not easy from the foregoing reasons. Further, the formed liposome easily formed a multi-layered membrane and the inclusion rate of the iodine compound was low. Means for allowing a water-soluble non-electrolyte to be efficiently included in a liposome include, for example, a reversed phase evaporation method and an ether injection method. In these means, however, organic solvents are used, producing problems of safety.

JP-A No. 2003-119120 discloses a method of preparing liposome-containing cosmetics or skin medicines for external use by using supercritical carbon dioxide, which is exemplified in the preparation of a skin medicine for external use occluding hydrophilic or hydrophobic medicinal components in a liposome. However, although examples of a water-soluble electrolyte are shown therein as a medicinal component, it is unclear whether a water-soluble non-electrolyte is efficiently included in a liposome using this method.

Even if inclusion of a contrast medium material is done well, problems such as its leaking-out over an elapse of time or the situation of the liposome itself becoming unstable must be taken into account. It is further pointed out that since a liposome introduced into an organism is almost always trapped in a reticuloendothelial system such as the liver or spleen, the intended effects cannot be achieved (Cancer Res., 43, 5328 (1983)).

### DISCLOSURE OF THE INVENTION

It is an object of the present invention to provide a contrast medium for X-ray diagnosis exhibiting efficient conveyance and high selectivity by including a contrast medium material in a liposome. Specifically it is an object of the invention to provide a radiographic contrast medium capable of detecting minute cancer tissue and exhibiting superior representation of tumors, in which an iodine compound is enclosed in liposomes without using a toxic organic solvent, and a preparation method thereof.

The above mentioned object is realized by the following constitution.

A radiographic contrast medium of the invention is characterized in that the medium comprises liposomes enclosing a water-soluble nonionic iodine compound, and the contrast medium is substantially free of a chlorine-containing solvent.

The contrast medium is characterized in that the liposome is prepared by a process of mixing a phospholipid with a supercritical carbon dioxide or a subcritical carbon dioxide and bringing a water-soluble nonionic iodine compound into contact with the phospholipid to form the liposomes.

The liposomes are preferably comprised substantially of unilamellar vesicles.

The iodine compound preferably contains at least one 2,4,6-triiodopheny group.

The liposomes preferably have an average vesicle size of 0.05 to 0.5 µm.

The average vesicle size is preferably 0.05 to 0.2 µm.

The average vesicle size is preferably 0.11 to 0.13m.

The liposomes preferably comprise a lipid membrane modified with a polyethylene glycol having 10 to 3500 oxyethylene units in an amount of 0.1% to 30% by weight, based on lipid forming the vesicles.

The liposomes preferably are those which has been filtered with a filter having a pore size of 0.1 to 0.4 µm.

The liposomes preferably include the iodine compound in a weight ratio of the iodine compound to liposome membrane lipid of 1 to 10.

The weight ratio of the iodine compound to liposome membrane lipid is 3 to 8.

The weight ratio of the iodine compound to liposome membrane lipid is 5 to 8.

The iodine compound included in the liposomes accounts for 5% to 30% by weight of a total iodine compound amount of the contrast medium.

The liposomes preferably comprise a lipid membrane and a water phase included inside the lipid membrane, the lipid membrane contains at least a compound selected from the group consisting of compounds containing a polyoxyalkylene oxide group and sterols and the water phase contains the iodine compound.

The liposomes preferably comprise a lipid membrane and a water phase included inside the lipid membrane, the lipid membrane contains a phospholipid modified with a polyalkylene oxide and the water phase contains the iodine compound.

The liposomes are vesicles comprising a lipid membrane and a water phase included inside the lipid membrane, the lipid membrane contains a block copolymer of polyethylene oxide and polypropylene oxide and the water phase contains the iodine compound.

The liposomes which comprise a lipid membrane and a water phase included inside the lipid membrane, are preferably dispersed in an aqueous medium, both of the water phase and the aqueous medium contain the iodine compound and an additive and each of an iodine compound concentration and an additive concentration is substantially the same in both of the water phase and the aqueous medium.

The additive preferably is a water-soluble amine type buffering agents or a chelating agent.

The amine type buffering agent preferably is trometamol.

The chelating agent preferably is EDTA Na₂-Ca.

The preparation method of the invention comprises mixing a phospholipid with a supercritical carbon dioxide or a subcritical carbon dioxide to form a mixture and bringing a water-soluble nonionic iodine compound into contact with the phospholipid to form a liposome comprised of vesicles including the iodine compound.

The method preferably comprises mixing a phospholipid with a supercritical carbon dioxide or a subcritical carbon dioxide to form a mixture, introducing an aqueous solution containing a nonionic iodine compound into the mixture and discharging the carbon dioxide to form a liposome comprised of vesicles including the iodine compound.

The carbon dioxide is preferably applied by a pressure of 50 to 500 kg/cm².

The temperature of the carbon dioxide preferably is 25 to 200 °C.

In the above mentioned method, the phospholipid and at least one compound, as a liposome membrane component, selected from a phospholipid modified with a polyalkylene oxide, a compound containing a polyoxyalkylene oxide group, a compound containing a polyethylene glycol group and a sterol are mixed, together with the phospholipid, with supercritical or subcritical carbon dioxide, an aqueous solution of the iodine compound is introduced thereto, thereafter, the carbon dioxide is discharged to form a liposomes enclosing the iodine compound, and the liposomes are filtered with a filter having a pore size of 0.1 to 0.4 µm.

In the above mentioned method, the phospholipid and at least one compound, as a liposome membrane component, selected from a phospholipid modified with a polyalkylene oxide and a sterol is mixed, together with the phospholipid, with supercritical or subcritical carbon dioxide, an aqueous solution of the iodine compound is introduced thereto, thereafter, the carbon dioxide is discharged to form a liposome enclosing the iodine compound, and the liposome is filtered with a filter having 0.1 to 0.4 µm pores to form the liposome which comprise a lipid membrane and a water phase included inside the lipid membrane, are dispersed in an aqueous medium, both of the water phase and the aqueous medium contain the iodine compound and the additive and each of an iodine compound concentration and an additive concentration is substantially the same in both of the water phase and the aqueous medium.

### DETAILED DESCRIPTION OF THE INVENTION

The present invention will be further described in detail with respect to contrast medium compounds, liposome and radiographic contrast medium.

### Contrast Medium Compound

Any ionic or nonionic water-soluble iodine compound capable of functioning as a contrast medium is usable in this inventions. Nonionic iodine compounds, which generally exhibit a lower osmotic pressure than ionic iodine compounds, are preferred. Specifically, water-soluble nonionic iodine compounds containing at least one iodophenyl group such as a 2,4,6-triiodophenyl group are preferred in this invention.

Specific examples of such a nonionic iodine compound include iohexol, iopentol, iodixanol, iopromide, iotrolan, iomeprol, N,N'-bis[2-hydroxy-1-(hydroxymethyl)-ethyl]-5-[(2-hydroxy-1-oxopropyl)-amino]-2,4,6-triiodo-1,3-benzene-dicarboxyamide (iopamidol) and metrizamide.

Further, examples of the nonionic iodine compounds include diatrizoic acid, diatrizoate sodium, meglumine diatrizoate, acetorizoic acid and its soluble salt, diprotrizo acid, iodamide, iodipamide sodium, meglumine iodipamide, iodohippuric acid and its soluble salt, iodomethamic acid, iodopyracet, iodo-2-pyrridone-N-acetic acid, 3,5-diiodo-4-pyridone-N-acetic acid (iodopyracet), diethylammonium salts of the foregoing acids, iothalamic acid, metrizoic acid and its salt, iopanoic acid, iocetamic acid, iophenoic acid and its soluble salt, sodium thyropanoate, sodium iopodate and other similar iodinated compounds.

These iodine compounds may be used alone or in combination. The iodine compounds usable in this invention are not limited to the foregoing exemplified compounds and include not only a free form but also its salt and hydrate.

As iodine compounds suitable for radiographic contrast medium of this invention are preferred iomeprol, iopamidol, iotrolan and iodixanol, which exhibit high hydrophilicity and a low osmotic pressure even at relatively high concentration. Specifically, dimeric nonionic iodine compounds such as iotrolan and iodixanol have advantages such that when prepared in the same iodine concentration, the prepared contrast medium has a lower overall molar quantity, resulting in a reduced osmotic pressure.

The concentration of the water-soluble iodine compound contained in the contrast medium according to this invention can be arbitrarily set based on factors such as properties of the contrast medium compound, the intended dosage route of a medicine and clinical guidelines. The quantity of the water-soluble iodine compound included in a liposome is typically 5% to 95% by weight, and preferably 5 to 70% by weight, based on total iodine compounds contained in the contrast medium. Specifically, to prevent unstabilization of the liposome including a capsulated iodine compound of the invention, the water-soluble iodine compound included in the liposome is usually 5% to 30%, preferably 5% to 25%, and more preferably 5% to 20% by weight, based on the total iodine compound. If the proportion of a water-soluble iodine compound included within liposome vesicles accounts for 5% to 25% by weight (preferably 5% to 20% by weight) of the total iodine compound contained in the contrast medium, effluent of the included iodine compound to the aqueous dispersion outside the vesicles, in which the residual 70% to 95% by weight (or 75% to 95% by weight) exists, can be substantially ignored, Accordingly, encapsulation of the iodine compound can prevent unstabilization due to the osmotic pressure effect of the liposome, leading to enhanced stability of contrast medium material.

### Liposome

In the radiographic contrast medium according to this invention, the foregoing contrast medium compounds are used in the form of enclosures in a liposome as a micro-carrier to efficiently and selectively convey the contrast medium compounds to the targeted region such as a targeted organ or tissue. The contrast medium can enhance its retention in the blood using a liposome with improved blood stability, thereby achieving efficient pharmaceutical conveyance and targeting. An improvement of closely related holding efficiencies such as the stabilized liposome structure and retention stability of included material and characteristics such as blood stability and blood retentivity are required to produce EPR (Enhanced Permeability and Retention) effect which is effective to achieve superior representation of tumors.

In the radiographic contrast medium of this invention, appropriate design of the vesicle size and bimolecular membrane of the liposome including a contrast medium compound can achieve targeting functions, in which both passive targeting and active targeting are taken into account. The former can control biological behavior through adjustment of vesicle size, lipid composition or charge of the liposome. Adjustment of the liposome vesicle size to a narrow range can be easily accomplished by the method to be described later. Design of the liposome membrane surface can be achieved by varying the kind and composition of phospholipids and included material to provide desired characteristics.

There should be also examined the introduction of active targeting which enables higher integrality and selectivity of the contrast medium. For example, introduction of a polymer chain of polyalkylene oxide or polyethylene glycol (PEG), which can control the guidance process to the targeted region, is extremely beneficial. A liposome suitable for the contrast medium of the invention is one in which the surface is modified with polyalkylene oxide or PEG to enhance blood retention and one which is not easily binged by reticuloendothelial cells such as liver. The contrast medium which does not reach cancerous tissue or the diseased region is externally discharged without being accumulated in the normal region, before causing adverse effects by the degradation of the liposome. This can be attained by optimal control of stability of a liposome in relation to the external discharge time in the design of the liposome. Iodine compounds as contrast medium material can be promptly discharged into urine via the kidney, thereby preventing adverse influences caused by internal retention in vain and delaying adverse effects.

Next, the design and preparation of the liposome used in the conveyance system of the radiographic contrast medium will be described.

A liposome is usually formed of a lipid bilayer. In general, a phospholipid and/or a glycolipid are preferably used as a constituent of the lipid layer.

Examples of a preferred neutral phospholipid in the liposome include lecithin and lysolecithin obtained from soybean or egg yolk, and their hydrogenation products or hydroxide derivatives. Further, examples of phospholipids include phosphatidylcholine, phosphatidylserine, phosphatidylinositol, phosphatidylglycerol, phosphatidylethanolamine, and sphingomyelin, which are derived from egg yolk, soybeans or other plants and animals, or are semi-synthetically obtainable; phosphatidic acid, dipalmitoylphosphatidylcholine (DPPC), distearoyl-phosphatidylcholine (DSPC), dimyristoylphosphatidylcholine (DMPC), dioleylphosphatidylcholine (DOPC), dipalmitoyl-phosphatidylglycerol (DPPG), distearoylphosphatidylserine (DSPS), distearoylphosphatidylglycerol (DSPG), dipalmotoyl-phosphatidylinositol (DPPI), distearoylphosphatidylinositol (DSPI), dipalmitoylphosphatidic acid (DPPA) and distearoyl-phosphatidic acid (DSPA), which are synthetically obtainable.

These phospholipids may be used alone or in two or more combinations. Two or more charged phospholipids are preferably used in combination of negative-charged phospholipids or in combination of positive-charged phospholipids in term of prevention of aggregation of the liposome. The combined use of a neutral phospholipid and charged phospholipid is preferably in a weight ratio of 200:1 to 3:1, more preferably 100:1 to 4:1, and still more preferably 40:1 to 5:1.

Examples of glyceroglycolipids include glycerolipids such as digalactosyldiglyceride and digalactosyldiglyceride sulfuric acid ester; sphingoglycolipids such as galactosylceramide, galactosylceramide sulfuric acid ester, lactosylceramide, ganglioside G7, ganglioside G6 and ganglioside G4.

In addition to the foregoing lipids, other substances may optionally incorporated as a constituent of the liposome membrane. For example, cholesterol, dihydrocholesterol, cholesterol ester, phytosterol, sitosterol, stigmasterol, campesterol, cholestanol, lanosterol and 2,4-dihydroxylanosterol are cited as a layer stabilizer. Further, sterol derivatives such as 1-0-sterolglucoside, 1-O-sterolmaltoside and 1-O-sterolgalactoside have been shown to be effective in stabilization of liposome, as described in JP-A No. 5-245357, and of the foregoing sterols, cholesterol is specifically preferred. Sterols are preferably used in an amount of 0.01 to 0.5 mol. and more preferably 0.05 to 0.1 mol per mol of phospholipid. An amount of less than 0.01 mol does not achieve stabilization by a sterol to enhance dispersibility of mixed lipids, and an amount of more than 0.5 mol inhibits liposome formation or results in unstable formation thereof.

A cholesterol included in the liposome membrane is capable of functioning as an anchor to introduce a polyalkylene oxide. Concretely, cholesterol which is included in the membrane as a liposome membrane constituent may optionally be linked via an anchor to a polyalkylene oxide group. A short chain alkylene group or oxyalkylene group can be used as an anchor. JP-A No. 9-3093 discloses novel cholesterol derivatives, in which various functional substances can be efficiently fixed at the top of a polyoxyalkylene chain, which can be employed as a liposome constituent.

Other additive compounds include, for example, negative charge-providing material, phosphoric acid alkyl esters such as diacetyl phosphate and positive charge-providing material, aliphatic amines such as stearylamine.

In this invention, a phospholipid or compound which contains a polyalkylene oxide (PAO) group (or polyoxyalkylene group, -(RO)ₙ-H , in which R is alkylene) or a similar group is used as a constituent of the liposome vesicular membrane to attain the desired purpose of the radiographic contrast medium. To overcome problems such as being trapped in a reticuloendothelial cell and problems regarding instability of liposome itself such as destruction or aggregation, there was attempted introduction of a polymeric chain, such as polyethylene glycol (PEG) chain (or polyoxyethylene group, - (CH₂CH₂O)ₙ-H) onto the liposome surface, as described in JP-A No. 1-249717 and FEBS letters 268, 235 (1990). Attachment of a polyalkylene oxide chain (or polyoxyalkylene chain) or a PEG chain onto the liposome vesicular surface can provide a new function to the liposome. For example, such a PEG-modified liposome can be expected to have an effect of becoming less recognizable from an immune system (so-called state of being stealthy). It was proved that a liposome having a hydrophilic tendency increased blood stability and thereby the concentration in blood can be stably maintained over a long period of time, as described in Biochim. Biophys. Acta., 1066, 29-36 (1991). JP-A No. 2002-37833 disclosed a technique in which a phospholipid modified with a polyalkylene oxide was incorporated into the liposome membrane to enhance blood retentivity of the liposome. Such a liposome was also shown to exhibit improved aging stability.

Employment of the foregoing properties can provide organ-specificity to the radiographic contrast medium of the invention. For example, since lipid components are easily accumulated in liver, the use of a liposome containing no PEG or trace amounts of PEG is desired to selectively provide contrast to the liver. Increasing the vesicle size to 0.2 µm or more results in increased possibility of being promptly incorporated by phagocytosis of liver Kupffer cells, leading to accumulation in the said site of the liver. In imaging liver cancer, since cancerous tissue has fewer Kupffer cells than normal tissue, incorporation of the contrast medium liposome becomes a less amount, forming clear contrast. It is similar in spleen.

On the contrary, in the case of imaging other organs, the liposome becomes a state of being stealthy by introduction of a PEG, becoming difficult to be gathers in the liver, therefore, the use of a PEG-modified liposome is recommended. Introduction of a PEG forms a hydration sphere, thereby stabilizing the liposome and enhancing blood retention. Functions can be adjusted by changing a length of oxyethylene units of a PEG and its introducing ratio. Polyethylene glycol having 10 to 3500 oxyethylene units is preferred as PEG. A PEG is preferably contained in an amount of 0.1% to 30% by weight, and more preferably 1% to 15% by weight, based on the lipid constituting the liposome.

PEG-modification of a liposome can be accomplished using commonly known techniques. For example, PEG linked to an anchor compound(e.g., cholesterol) is mixed with a phospholipid as a membrane constituent to form a liposome and the anchor compound may be allowed to be linked to activated PEG. Since the polyethylene glycol group introduced onto the liposome surface is unreactive with "functional material" to be described later, it is difficult to fix the functional material onto the liposome surface. Instead thereof, PEG, the top of which has been chemically modified is bonded to a phospholipid, which is included as a constituent for liposome to prepare liposome.

In place of the foregoing PEG may be introduced commonly known polyalkylene oxide groups, represented by general formula: -(AO)ₙ-Y, wherein AO is an oxyalkylene group having 2 to 4 carbon atoms, n represents a mean addition molar number and is a positive number of 1 to 2000; and Y is a hydrogen atom, an alkyl group or a functional group. Examples of an oxyalkylene group having 2 to 4 carbon atoms include an oxyethylene group, oxypropylene group, oxytrimethylene group, oxytetramethylene group, oxy-1-ethylethylene group and oxy-1,2-dimethyletthylene group. These oxyalkylene groups are ones which can be obtained by addition polymerization of alkylene oxide, such as ethylene oxide, propylene oxide, oxetane, 1-butene oxide, 2-butene oxide or tetrahydrofuran. Positive number n is 1 to 2,000, preferably 10 to 500, and more preferably 20 to 200. When n is 2 or more, plural oxyalkylene groups may be the same or differ. In the latter case, differing oxyalkylene groups may be in a random form or in a block form. To provide hydrophilicity to an oxyalkylene group, ethylene oxide alone is preferably addition-polymerized, in which n is preferably 10 or more. In cases when different alkylene oxides are addition-polymerized, it is desirable that at least 20 mol% (preferably at least 50 mol%) of ethylene oxide is addition-polymerized. To provide lipophilicity to an oxyalkylene group, it is preferred to increase the molarity of alkylene oxide(s) other than ethylene oxide. For example, a liposome containing a block copolymer of polyethylene oxide and polypropylene oxide (or polyethylene oxide-block-polypropylene oxide) is one preferred embodiment of this invention. Y is a hydrogen atom, an alkyl group or a functional group. The alkyl group is an aliphatic hydrocarbon group having 1 to 5 carbon atoms, which may be branched. The functional group is to attach functional material such as sugar, glycoprotein, antibody, lectin and a cell adhesion factor to the top of a polyalkylene oxide group and examples thereof include an amino group, oxycarbonylimidazole group and N-hydroxysuccinimide.

The liposome anchoring a polyalkylene oxide chain, to the top of which the foregoing functional material is bonded, not only exhibits effects due to introduction of a polyalkylene oxide group but also gives full play of functions of the functional material, for example, a function as a recognition element, such as directivity to a specific organ (namely organotropism) and cancer tissue directivity.

A phospholipid or compound which contains a polyalkylene oxide group can be used alone or in combinations thereof. The content thereof preferably is 0.001 to 50 mol%, more preferably 0.01 to 25 mol%, and still more preferably 0.1 to 10 mol%, based on the total amount of liposome membrane forming components. A content of less than 0.001 mol% results in reduced expected effects.

Commonly known techniques can be employed to introduce a polyalkyleneoxide chain to a liposome. For example, an anchor (e.g., cholesterol) is mixed with a phospholipid as a constituent of the membrane to form a liposome and an activated polyalkylene oxide is allowed to be attached to the anchor. In such a method, it is necessary to perform a multistage chemical reaction on the liposome membrane surface after formation of a liposome, which limits the introducing amount of the intended functional material or results in contamination of reaction by-products or impurities, producing problems such as increased damage to the liposome membrane.

As a preferred preparation method, on the other hand, phospholipid polyalkylene oxide (PEO) derivatives are included in advance in raw material, such as phospholipids to form liposome. There were proposed modified phospholipids suitable for such a method, which were represented by the following formula, as described in JP-A No. 7-165770: 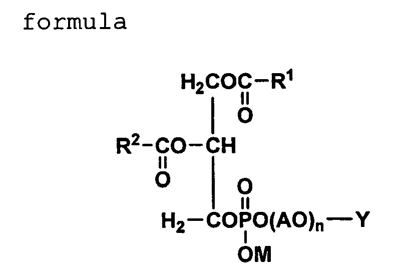 wherein R₁ and R₂ are each an alkyl group having 2 to 29 carbon atoms; M is a hydrogen atom or an alkali metal atom such as sodium or potassium; AO n and Y are each the same as defined in the foregoing.

Specific examples thereof include polyethylene oxide (PEO) derivatives of a phosphatidylamine and the like, such as distearoylphosphatidyldiethanolamine polyethyleneoxide (DSPE-PEO). Further, JP-A No. 2002-37883 discloses extremely purified, polyalkylene oxide-modified phospholipid to prepare a water-soluble polymer-modified liposome exhibiting enhanced blood retentivity. It is also disclosed that the use of a polyalkylene oxide-modified phospholipid having a relatively low monoacyl content in the preparation of liposome leads to superior aging stability of liposome dispersions.

There have been proposed various methods for preparing liposomes. Different methods often finally lead to liposomes extremely differing in form and characteristics, as described in JP-A No. 6-80560. Therefore, a method is optimally chosen in accordance with the form or characteristics of a desired liposome. In general, a liposome is prepared by dissolving lipid components such as phospholipid, sterol and lecithin, almost without an exception, in organic solvents such as chloroform, dichloromethane, ethyl ether, carbon tetrachloride, ethyl acetate, dioxane or tetrahydrofuran (THF), in which specifically, chlorine-containing solvents are often used. After the volatile material is distilled away under reduced pressure, the lipid mixture is dispersed in a buffer solution containing a prescribed amount of an inclusion material. Subsequently, the dispersion is stirred for a few hours, causing formation of a liposome and then, a part of the dispersion (containing the inclusion material) is included in liposome vesicles. Next, the size of the liposome and the viscosity of the dispersion are reduced by subjecting the dispersion to an ultrasonic treatment, by the use of surfactants, or by other methods. Such liposome products necessarily contain organic solvent. It is difficult to completely remove residual organic solvents, specifically, chlorinated organic solvents, which is of concern for adverse effects on organisms.

To prepare liposomes used in this invention, a preparation method using supercritical or subcritical carbon dioxide, in which any organic solvent, specifically, any chlorinated organic solvent is not used, is employed to avoid the foregoing problems. Carbon dioxide is suitable because it exhibits a critical temperature of 31.1 °C and a critical pressure of 73.8 bar and can be relatively easily handled, is an inert gas and non-toxic to the human body even when being remained, and a high-purity liquid is inexpensively and easily available. The liposome prepared by this method has preferred characteristics and advantages to include iodine compounds used as a radiographic contrast medium, as described later.

To prepare a liposome using supercritical or subcritical carbon dioxide, it is necessary to dissolve, disperse or mix the foregoing lipid membrane constituents in carbon dioxide in the supercritical state (including subcritical state). The thus supercritical carbon dioxide is easily permeable into material and exhibits superior solubility. In this case, the use of one or more alcohols as an auxiliary solvent, such as a lower alcohol, glycol or glycol ether is preferred to enhance the solubility of the foregoing lipid membrane constituents. For example, it is preferred to use alcohols as an auxiliary solvent, in an amount of 0.1% to 10%, and more preferably 1% to 8% by weight, based on supercritical carbon dioxide. Of the auxiliary solvents, ethanol is preferred in terms of safety and easy removal.

The pressure suitable for carbon dioxide in the supercritical state (including the subcritical state) is 50 to 500 kg/cm² and preferably 100 to 400 kg/cm². The temperature suitable for carbon dioxide in the supercritical state (including the subcritical state) is 25 to 200 °C, preferably 31 to 100 °C, and more preferably 35 to 80 °C. The supercritical state (including the subcritical state) of carbon dioxide is achieved by optimally selecting and combining a pressure and a temperature which fall within the foregoing range.

The suitable preparation of a liposome used for the radiographic contrast medium of this invention is conducted in the following manner. Liquid carbon dioxide is added in the presence of liposome membrane constituents and brought to the supercritical state or subcritical state under the suitable pressure and temperature described above. At least one compound, as a membrane lipid, selected from a polyalkylene oxide-modified phospholipid, polyalkylene oxide-containing compound, a polyethylene glycol-containing compound and sterols may be mixed with the foregoing phospholipid. In this case, the auxiliary solvents described above may optionally be used. Subsequently, an iodine compound and optionally, a solution containing additives such as a pharmaceutical preparation aid are continuously added to form a water/carbon dioxide emulsion. In this emulsion, it is contemplated that the lipid component is dispersed in the form of micelles, while being aggregated or separated. After stirring is continued for a while and the micelle-containing emulsion is stabilized, water is further continuously added until the carbon dioxide phase and the water phase are separated. It is supposed that increasing the water phase causes phase transition and excessive carbon dioxide is separated from the carbon dioxide/water emulsion via two phase systems of water/carbon dioxide emulsion plus carbon dioxide/water. The liposome is contemplated to be transferred to the water phase and evacuating the system to discharge carbon dioxide forms an aqueous dispersing solution in which liposome vesicles occluding the iodine compound are dispersed. In this case, the iodine compound may be contained in a water phase (external water phase) outside the vesicles besides the water phase in the interior of the liposome vesicles. Since the foregoing aqueous solution is included in the interior of the liposome vesicles, the iodine compound is present not only in the external water phase but also mainly in the water phase in the interior of the liposome vesicles, which is in the state of so-called endocyst. Further, the liposome is allowed to pass through a porous filter having a pore size of 0.1 to 0.4 µm. Subsequently, via the pharmaceutical preparation process such as a sterilization treatment and packaging, the radiographic contrast medium of this invention is prepared.

The preparation of liposome using supercritical or subcritical carbon dioxide has been proved to be high in liposome formation rate, inclusion percentage of including material and remaining ratio of included material in liposome, as described in JP-A No. 2003-119120. Further, the foregoing method of this invention, which is applicable even on an industrial scale and enables effective inclusion of a nonionic water-soluble material in a liposome, substantially without using any organic solvent, is useful for preparation of the radiographic contrast medium of this invention. Specifically, the contrast medium of this invention is substantially free of a chlorine-containing solvent. Herein, the expression "substantially free of a chlorine-containing solvent" means that the contrast medium contains chlorine-containing solvent(s) in an amount of not more than 10 µg per liter of the contrast medium. Examples of a chlorine-containing solvent include dichloromethane, dichloroethane, trichloromethane and trichloroethylene.

The liposome relating to this invention is comprised substantially of unilamellar vesicles, that is, a unilamellar vesicle formed of a single phospholipid bilayer. The expression, "substantially" means that a vesicle is made up of a phospholipid bilayer, the replica of which is recognized nearly as a single layer in transmission electron microscopic observation using a freeze fracture replica technique. Thus, when observing the imprint of particles remaining in the carbon film, one having no difference in level is judged as a unilamellar vesicle and one having two or more differences is a multilamellar vesicle. In this invention, such unilamellar vesicles are contained preferably in an amount of at least 80%, and more preferably at least 90%, based on the total liposome amount, i.e., the total amount of vesicles contained in the radiographic contrast medium.

Unilamellar vesicles can be efficiently prepared using the foregoing supercritical carbon dioxide as a solvent for lipids and by a phase separation method using water. On the contrary, in conventional methods for preparation of liposome, a liposome comprised of multilamellar vesicles (MLV), that is, multilamellar vesicles often account for a fairly high proportion. Accordingly, operations such as exposure to ultrasonic or filtering through given-sized pores are required to raise the proportion of unilamellar liposome. Unilamellar vesicles have advantages such that an amount of added liposome or a given lipid amount is usually less than that of multilamellar vesicles.

A unilamellar vesicle, specifically, a large unilamellar vesicle (LUV) advantageously has a larger inclusion capacity than a multilamellar vesicle. A liposome used in the radiographic contrast medium is between LUV having a size of 0.2 to 1.0 µm and SUV (small unilamellar vesicles) of less than 0.05 µm. Accordingly, the retention volume exceeds the SUV and the trapping efficiency of an iodine compound, specifically, a water-soluble iodine compound, in other words, inclusion efficiency becomes superior, as described later. Further, differing from MLV or LUV, this vesicular liposome does not rapidly disappear from the bloodstream due to incorporation into reticuloendothelial cells.

However, even a unilamellar vesicle exhibiting superior inclusion efficiency of an iodine compound lowers its stability when the weight of an included iodine compound is relatively excessive. It was observed that it exhibited a tendency of being weak for rapid change of ionic strength. The liposome used in the contrast medium of the invention was adjusted to a relatively small vesicle size and incorporation of sterols in the vesicle membrane or incorporation of a phospholipid or compound containing polyalkylene oxide led to enhanced stability of the lipid membrane. As a result, it was proved that such liposome was resistant to salt shock.

The vesicular particle size (hereinafter, also denoted simply as vesicle size) of the liposome and distribution thereof are closely correlated with enhanced blood retention property, targeting ability and conveyance efficiency which are aimed in this invention. The vesicle size can be determined in such a manner that a dispersion containing liposome vesicles including an iodine compound is frozen and fractured, following which carbon is vapor-deposited onto the fractured interface and the deposited carbon is observed by an electron microscope (freeze fracture TEM method). In this invention, the average vesicle size refers to an arithmetic average value of 20 vesicles observed. The vesicle size can be adjusted by formulation or control of processing conditions. For example, increasing the supercritical pressure described earlier results in a liposome of a decreased vesicle size. Filtration may be conducted using polycarbonate film to allow the vesicle size distribution to fall within a narrower range. In this regard, a liposome of unilamellar vesicles having an optimal average vesicle size of not more than 0.5 µm can be efficiently obtained by passing it through an extruder installed with a filter of 0.1 to 0.4 µm pore size. Introduction of such an extruding operation, in addition to the foregoing sizing operation, has advantages such as adjustment of a concentration of an iodine compound existing outside the liposome, change of liposome dispersing solution and removal of unintended material.

Sizing of the liposome particles is important to enhance active targeting capability of the liposome. For example, Japanese Patent No. 2619037 discloses that unfavorable retention in the lung capillary can be avoided by removal of liposome of 3 µm or more. However, liposome of 0.15 to 3.0 µm does not necessarily exhibit tumor directivity.

The average vesicle size of a liposome used in the contrast medium of the invention is usually 0.05 to 0.5 µm, preferably 0.05 to 0.3 µm, more preferably 0.05 to 0.2 µm, and still more preferably 0.05 to 0.13 µm. The average vesicle size can be adjusted in accordance with the objective of X-ray imaging. For example, when 0.11 to 0.13 µm is preferred for the purpose of selective imaging of a tumor region. Adjusting the average liposome vesicle size so as to fall within the range of 0.1 to 0.2 µm (preferably 0.11 to 0.13 µm) enables selective concentration of the contrast medium to cancerous tissue. This is known as the EPR effect. The pore on the vascularized wall of solid cancerous tissue is abnormally larger than a 0.03 to 0.08 µm pore size of capillary wall fenestra of normal tissue, so that even a large molecule of ca. 0.1 to ca. 0.2 µm leaks through the vascular wall. Thus, the EPR effect is due to permeability of the vascularized wall of cancerous tissue which is higher than the microvascular wall of normal tissue.

Since lymphatic vessels do not sufficiently develop around cancerous tissue, the contrast medium leaking through vascular walls does not return to the blood vessel and remains there for a relative long time. The EPR effect is a passive conveyance employing the bloodstream so that enhanced blood retention is required to effectively develop the effect. Thus, contrast medium particles (or liposome vesicular particles including an iodine compound) are required to be retained in blood and to pass many times through blood vessels near cancer cells. The radiographic contrast medium of this invention, which does not contain any particularly large particle, does not easily become a target for trapping by reticuloendothelial cells. Liposome vesicles, which are in a form similar to an erythrocyte and also behaves similarly to an erythrocyte, are retained long in blood and not promptly discharged via the kidneys and further not binged by reticuloendothelial cells when being masked. The EPR effect necessarily enhances transfer of the contrast medium compound to the targeted organ or tissue and achieves selective concentration and accumulation in cancerous tissue of the contrast medium. A rise of the accumulation ratio of oncocyte/normal cell enhances contrast performance of the contrast medium. Improved tumor visualization enables finding-out a micro-metastatic cancer which has up to now been difficult to detect.

### Radiographic Contrast Medium

In the radiographic contrast medium of this invention, the retention efficiency of contrast medium material included in the liposome is improved through enhancement of structure stability of the liposome and enhancement of retention stability of the contrast medium material.

The contrast medium may contain an iodine compound and at least one auxiliary additive (pharmaceutical preparation aid) preferably in the water phase inside the liposome membrane and in an aqueous medium in which the liposome vesicles are dispersed, in which the respective concentrations preferably are substantially the same between inside and outside the membrane. The expression, substantially the same means concentrations being nearly the same. An auxiliary additive refers to a compound which is to be added together with the contrast medium material and various substances can be employed based on techniques for preparing contrast mediums. Specific examples thereof include physiologically acceptable buffering agents, chelating agents such as disodium calcium ethylenediaminetetraacetate (also denoted as EDTA disodium calcium or EDTA Na₂-Ca) or disodium ethylenediaminetetraacetate (also denoted EDTA disodium or EDTA Na₂) and optionally, an osmotic pressure-adjusting agent, a stabilizer, an antioxidant such as α-tocopherol, and a viscosity adjusting agent. Water-soluble amine type buffering agents and chelating agents are preferably included. As a pH buffering agent, amine-type buffering agents and carbonate type buffering agents are preferred, of which amine type buffering agents are more preferred and trometamol (also denoted as tromethamine or 2-amino-2-hydroxymethyl-1,3-propanediol) is specifically desirable. Of chelating agents preferable is EDTANa₂-Ca. An aqueous medium refers to a solvent comprised basically of water capable of dissolving iodide compounds or auxiliary additives. An aqueous solution, other than the water phase inside the liposome membrane (or an included aqueous solution), namely, an aqueous medium in which the liposome vesicles are dispersed, also contains the iodine compound and auxiliary additive(s), such as a water-soluble amine type buffering agent or chelating agents. Accordingly, no difference in osmotic pressure is caused between inside and outside of the membrane, whereby structure stability of the liposome is maintained.

There has been revealed the amount of an iodine compound conveyed to a targeted organ to provide a prescribed contrast performance in radiography, as described in Japanese Patent No. 2619037. In cases when an iodine compound is included in a microcarrier such as a liposome, in addition to the conveyance efficiency and retention stability of contrast medium material, the weight of vesicular membrane lipid must be taken into account. An increase of the membrane lipid weight increases the viscosity of the contrast medium. The amount of an iodine compound included in the liposome, which is contained in an aqueous solution included in liposome vesicles, is preferably is 1 to 10, more preferably 3 to 8, and still more preferably 5 to 8, in terms of the weight ratio of the iodine compound included in the vesicles to the lipid forming the vesicular membrane.

A weight ratio of the iodine compound included in the liposome of less than 1 necessitates injection of a relatively large amount of the lipid, resulting in lowered conveyance efficiency of the contrast medium material. According to Japanese Patent No. 2619037, even a weight ratio of 1 was described to be a relatively high value in light of the technical level at that time. Since the viscosity of the radiographic contrast medium depends on the lipid amount of the liposome, the unilamellar liposome which exhibits enhanced retention volume and inclusion efficiency is apparently superior. On the contrary, when the weight ratio of the included iodine compound to the lipid of the liposome membrane exceeds 10, the liposome becomes structurally unstable and diffusion or leakage of the iodine compound from the liposome membrane is unavoidable during storage or even after being injected into the organism. Further, published Japanese translations of PCT international publication for patent application No. 9-505821 described that even if a 100% inclusion is achieved immediately after liposome dispersion is prepared and separated, the included ingredients decrease in a short time by unstabilization effects due to osmotic pressure.

If the radiographic contrast medium of the invention is dosed in a pharmaceutical solution of 10 to 300 ml, the iodine content is 100 to 50 mg I/ml, and preferably 150 to 300 mgI/ml. The viscosity of the pharmaceutical solution is not more than 6 cPa, and preferably 0.9 to 3 cPa at 37 °C.

In one preferred embodiment, the radiographic contrast medium which improves representation of the tumor and contrast medium retention stability of the liposome, comprises a liposome having a lipid membrane, the liposome contains a compound containing a polyalkylene oxide group and/or sterols in the lipid membrane, and an iodine compound is contained in the water phase included inside the lipid membrane. Further, said liposome is a unilamellar one, which is comprised of unilamellar vesicles of a average vesicle size of 0.05 to 0.2 µm, and which contains an iodine compound at a weight ratio of iodine compound to lipid membrane of 3 to 8, and the iodine compound included in the liposome is in an amount of 5 to 25% by weight, based on the total amount of iodide compound.

The contrast medium is included in the liposome in the form of an isotonic solution or suspension so that the liposome is stably retained in the human body. Water or buffer solutions such as tris-hydrochloric acid buffer, phosphoric acid buffer or citric acid buffer are usable as a medium of such a solution or suspension. The pH of the foregoing solution or suspension is preferably 6.5 to 8.5, and more preferably 6.8 to 7.8 at room temperature. In the case when the radiographic contrast medium is an iodine compound containing polyhydroxyl groups, a preferred buffer is one having a negative temperature coefficient, as described in U.S. Patent No. 4,278,654. Amine type buffers have properties meeting such a requirement and specifically preferred one is TRIS. Such a type of buffer exhibits a relative low pH at an autoclave temperature, which increases stability of the contrast medium in the autoclave and the pH returns to a physiologically allowable range at room temperature. In this case, there is no restriction of reducing the liposome vesicle size, such as filtration sterilization. Accordingly, it is very convenient to sterilize the liposome product in an autoclave to prepare an aseptic contrast medium for use in injectors, whereby storage stability can also be maintained. Liposome to which autoclave sterilization is not applicable may be subjected to filtration sterilization.

An isotonic solution or suspension is prepared by allowing a contrast medium material to be dissolved or suspended in a medium at a concentration giving an isotonic solution. For example, in case when an isotonic solution cannot be prepared with a contrast medium compound alone, due to its low solubility, a nontoxic water-soluble material, such as salts such as sodium chloride or saccharides, e.g., mannitol, glucose, saccharose and sorbitol may be added to form an isotonic solution or suspension.

The radiographic contrast medium of this invention is not given orally to a person but through intravascular dosage, preferably through intravenous dosage, as injection or dripping, followed by exposure to X-rays for imaging. The dose is the same as conventional iodine type contrast mediums. The total amount of iodine inside the liposome or the total amount of iodine inside and outside the liposome may be at the same level as conventional doses.

The radiographic contrast medium of this invention contains an iodine compound in a water phase included inside the liposome membrane and in the aqueous medium in which the liposome is dispersed, and the respective concentrations of the iodine compound are substantially the same between inside and outside of the membrane. This means that the identical iodine compound is concurrently present and exist in different forms. Such a state of the contrast medium material can exhibit the following advantages in diagnostic examination. According to the use of the radiographic contrast medium of this invention, the difference in diffusion time in the body between non-capsulated contrast medium material and one which is encapsulated in the liposome gives images differing in distributive behavior with the elapse of time, which provides useful diagnostic information.

In practical diagnostic examinations, the combined use of the contrast medium of this invention and a computer tomography apparatus is expected to effectively display further enhanced imaging performance. In computer tomography diagnosis of liver tumor, for example, the contrast medium material which is not encapsulated into the liposome, freely passes through the liver sinusoid and reaches liver parenchyma cells immediately after dosage, raising the image density of healthy liver tissue, which is rapidly lowered. This density lowering is compensated for by the successive reinforcement from the liposome contrast medium, whereby the contrast medium material can be maintained at a high concentration over a long period of time. Freed non-capsulated contrast medium material differs in distribution, which enables detailed analysis of the tissue state.

The radiographic contrast medium comprises liposomes comprising unilamellar vesicles having relatively uniform particle diameters, wherein an alkylene oxide chain and/or a sterol is contained in the lipid membrane and a water-soluble iodine compound is included in the water phase, leading to improvements of stability of the liposome structure and retention stability of the included material.

Further, the radiographic contrast medium of this invention comprises unilamellar liposome vesicles having relatively uniform particle diameters, wherein an iodine compound and auxiliary additive are contained both in the water phase inside the liposome membrane and the aqueous medium in which the liposome is dispersed, and the respective concentrations are substantially the same between inside and outside the membrane, thereby achieving improvements of stability of the liposome structure and retention stability of the included material.

Furthermore, the radiographic contrast medium of this invention exhibits superior blood retention and displays an EPR effect, resulting in selective and concentrated accumulation into the intended diseased region or tissue, specifically cancer tissue. After imaging, the iodine compound is water-soluble and readily discharged externally. Moreover, the difference in diffusion time in the body between non-capsulated contrast medium material and one which is encapsulated in the liposome gives images differing in distributive behavior with the elapse of time, which provides information useful in diagnosis.

Preparation of the foregoing liposomes introduces the method of dissolving a phospholipid or the like in supercritical carbon dioxide, in which the use of toxic solvents, specifically, highly toxic chlorine-containing solvents is not needed.

The radiographic contrast medium is usable in small amounts, compared to conventional radiographic contrast mediums, resulting in reduced toxicity and adverse effect and leading to reduced load onto the patient.

### EXAMPLES

The present invention will be further described based on examples but are by no means limited to these.

### Form and Liposome Vesicle size of Liposome

The liposome particle (or vesicle) size and the structure of liposomes contained in the contrast medium were determined in the freeze fracture method using a transmission electron microscope (TEM). Thus, a liposome dispersion was rapidly frozen with liquid nitrogen and fractured in the frozen state to expose the internal structure of the liposome. Then, carbon was vapor-deposited onto the fractured interface and the carbon deposit film was observed by a transmission electron microscope.

The vesicular particle size of the liposome was represented by an arithmetic average value of 20 particle diameters. With respect to the structure of liposome particles, when observing the imprint of particles remaining in the carbon film, one having no difference in level was judged as a unilamellar vesicle and one having two or more differences was a multilamellar vesicle. When at least 80% of 20 observed vesicular particles of a liposome was accounted for by a unilamellar structure, the liposome was judged as uniform vesicles (or a unilamellar liposome).

### Determination of Iodine Compound

A liposome dispersion was dialyzed with an isotonic sodium chloride solution. After completion of the dialysis, ethanol was added thereto to degrade the liposome and the amount of an iodine compound included in the liposome vesicles was determined in the absorptiometry.

### Example 1

Dipalmitoylphosphatidylcholine (DPPC) and carbon dioxide were added together with ethanol into a stainless steel autoclave and stirred with maintaining the autoclave at 60 °C and 300 kg/cm² to dissolve the DPPC in supercritical carbon dioxide. Iomeprol solution was continuously added using a metering pump, while stirring the supercritical carbon dioxide solution. The iomeprol solution was prepared in such a manner that 816.5 mg of iomeprol was dissolved in water for injection with heating, ascorbic acid was added in an amount of 20 mM and 1 mg of tromethamol was further added; the pH was adjusted to a physiological pH and finally, water for injection was added to make up 1.0 ml. Thereafter, the autoclave was evacuated to discharge carbon dioxide and a dispersion of liposomes containing iomeprol was obtained. The obtained dispersion was put into a glass vial and subjected to autoclave sterilization at 121 °C for 20 min to obtain a contrast medium.

The vesicular particle size of the liposome contained in the contrast medium was determined in the freeze fracture method using a transmission electron microscope (TEM). The average vesicle size of the liposome was 0.13 µm and the liposome was comprised of unilamellar vesicles.

### Example 2

The contrast medium obtained in Example 1 was diluted with an isotonic glucose solution to a concentration of 50 mg iodine/ml. When this solution was given to a rat by an intravenous injection, concentration to the liver was observed in radiography. It was noted that the imaging level (or image contrast) in the liver decreased in parallel to imaging levels of all other organs over an elapse of time, and almost all of the iomeprol was discharged into urine.

### Example 3

The contrast medium obtained in Example 1 was diluted with an isotonic glucose solution to a concentration of 50 mg iodine/ml. This solution was injected into a vein of a rat having a large number of transferred liver cancer cells. The transferred tumor region exhibited a high imaging level (high image contrast), in which tumors of 5mm in diameter were observed. Lowering of the image contrast in the tumor region was delayed over an elapse of time, compared to imaging levels of other organs.

### Example 4

Contrast mediums were prepared similarly to Example 1, provided that the pressure and temperature were adjusted so as to form a liposome having average liposome vesicle sizes of 0.07 µm, 0.18 µm and 0.22 µm.

### Example 5

A contrast medium was prepared similarly to Example 1, except that 40 g of a PEG having 2000 oxyethylene units was further added. The average liposome vesicle size was 0.12 µm.

### Example 6

Contrast mediums prepared in Examples 4 and 5 were each evaluated similarly to Example 2. The contrast medium prepared in Example 4 was radiographically observed to be specifically concentrated into the liver after injection. The contrast medium prepared in Example 5 was less concentrated into the liver than that of Example 4.

### Example 7

Contrast mediums prepared in Examples 4 and 5 were each evaluated similarly to Example 3. The contrast medium containing the liposome having an average liposome vesicle size of 0.07 µm was faster in lowering of the imaging level (image contrast) in the tumor region than the contrast medium of Example 3. The contrast medium containing the liposome having an average liposome vesicle size of 0.18 µm was delayed in lowering of the imaging level in the tumor region, compared to the contrast medium of Example 3. The contrast medium containing the liposome having an average liposome vesicle size of 0.22 µm exhibited a markedly lowered imaging level in the tumor region, compared to the contrast medium of Example 3. It was further proved that the contrast medium prepared in Example 5 exhibited a higher image contrast in the tumor region than the contrast medium of Example 3 and lowering of the imaging level was delayed.

### Comparative Example 1

A contrast medium was prepared in accordance with conventional procedure, provided that a dispersion containing a liposome was prepared by dissolving a phospholipid in an organic solvent instead of supercritical carbon dioxide. It was shown that the thus prepared liposome was not a unilamellar liposome. As a result of evaluation similar to Example 3, it was proved that the imaging level in the tumor region was markedly lowered, compared to the contrast medium of Example 3.

As apparent from the foregoing results, it was proved that a radiographic contrast medium prepared dissolving phospholipid in supercritical carbon dioxide exhibited superior imaging of cancer to conventional contrast medium prepared by dissolving phospholipid in organic solvents.

### Example 8

A mixture of 0.04 g of dipalmitoylphosphatidylcholine (DPPC), 1.2 mg of Pluronic F-88 (block copolymer of polyethylene oxide and polypropylene oxide, available from ADEKA Co.) and 0.9 g of ethanol was charged into a stainless steel autoclave and the autoclave was internally heated to 60 °C, subsequently, 13 g of liquid carbon dioxide was added thereto. The pressure inside the autoclave was raised from 50 kg/cm² to 200 kg/cm² and the autoclave was internally stirred to allow the DPPC to be dissolved in supercritical carbon dioxide. To this supercritical carbon dioxide solution was continuously added with stirring a solution which contained 647 mg/ml of iohexol (having an iodine content of 300 mg/ml), 1.21 mg/ml of tromethamol and 0.1 mg/ml of edetate calcium disodium and which was also adjusted to a pH of ca. 7 with hydrochloric acid or sodium hydroxide. Thereafter, the inside of the autoclave was evacuated to discharge carbon dioxide, whereby a dispersion of liposomes containing iohexol was obtained. The same operation was repeated a few times and the obtained dispersion was heated to 60 °C and subjected to pressure filtration using a 0.1 µm cellulose type filter, produced by Advantech Co. Sizes of liposome vesicles obtained through such pressure filtration were 0.15 µm or less. The thus obtained contrast medium was designated as Sample 1. The average liposome vesicle size was 0.12 µm and the liposome was substantially comprised of unilamellar vesicles. The weight ratio of the iodine compound included in vesicles to the vesicle membrane lipid was 5.6.

### Example 9

Similarly to Example 8, a liposome dispersion was prepared by subjecting to pressure filtration using a 0.1 µm cellulose type filter (produced by Advantech Co) and designated as Sample 2, except that the pressure was adjusted to a higher pressure side, the evacuation speed was controlled and the amount of the used phospholipid was varied. It was proved from transmission electron microscopic observation using the freeze fracture technique that the liposome of Sample 2 was comprised substantially of unilamellar vesicles. The liposome vesicle size is shown in Table 1.

### Example 10

Similarly to Example 8, a liposome dispersion was prepared by subjecting it to pressure filtration using a 0.1 µm cellulose type filter (produced by Advantech Co.) and designated as Sample 3, except that the pressure was raised, the evacuation speed was controlled and the amount of the used phospholipid was varied. From transmission electron microscopic observation using the freeze fracture technique, it was proved that the liposomes of Sample 3 were comprised substantially of unilamellar vesicles. The liposome vesicle size is shown in Table 1.

### Example 11

Similarly to Example 8, a liposome dispersion was prepared by subjecting it to pressure filtration using a 0.1 µm cellulose type filter (produced by Advantech Co.) and designated as Sample 4, except that the pressure was raised, the evacuation speed was controlled and the amount of the used phospholipid was varied. It was proved, from transmission electron microscopic observation using the freeze fracture technique, that the liposome of Sample 4 was substantially comprised of unilamellar vesicles. The liposome vesicle size is shown in Table 1.

### Example 12

Similarly to Example 8, a liposome dispersion was prepared by subjectingit it to pressure filtration using a 0.2 µm cellulose type filter (produced by Advantech Co.) and designated as Sample 5, except that the pressure was raised, the evacuation speed was controlled and the amount of the used phospholipid was varied. It was proved, from transmission electron microscopic observation using the freeze fracture technique, that the liposomes of Sample 5 were comprised substantially of unilamellar vesicles. The vesicular particle size is shown in Table 1.

### Comparative Example 2

A dispersion containing a liposome was prepared in a conventional manner, provided that a phospholipid was dissolved in organic solvents, instead of supercritical carbon dioxide and the amount of the used phospholipid was varied. Thereby, two kinds of radiographic contrast mediums were prepared (Samples 6 and 7). The thus prepared liposome was not one comprised of unilamellar vesicles. The vesicle size is shown in Table 1.

### Example 13

A cell suspension of VX2 calcinorma was hypodermically implanted into a rabbit. Two weeks after implantation, the contrast medium obtained in Example 8 was given by an intravenous injection and then radiographically observed. Although the Imaging level (specifically image contrast) decreased with the elapse of time, a decrease of the imaging level or image contrast in the implant region was delayed.

Contrast medium samples prepared in Example 9 to 12 (Samples 2 to 5) and contrast medium Sample 6 prepared in the Comparative Example 2 were similarly evaluated. Further, a contrast mediums (Sample 8, Comparative Example 3) which were prepared in accordance with sample 2B in Example 2 of Japanese Patent No. 2619037, were also evaluated. Results thereof are shown in Table 1. Imaging level of cancer was evaluated based on the following criteria:
A: a decrease of the image contrast in the implant region was apparently delayed compared to other regions,
B: a decrease of the image contrast in the implant region was slightly delayed compared to other regions,
C: a decrease of the image contrast in the implant region was almost the same as that of other regions,
D: an increase of the image contrast in the implant region
was little.

**Table 1**

| Sample No. | Average Vesicle Size (µm) | Weight Ratio of Included Iodine*¹ | Imaging Level of Cancer | Remarks |
|---|---|---|---|---|
| 1 | 0.12 | 5.6 | A | Example 8 |
| 2 | 0.12 | 3.4 | B | Example 9 |
| 3 | 0.10 | 4.6 | B | Example 10 |
| 4 | 0.08 | 3.4 | B | Example 11 |
| 5 | 0.13 | 5.8 | A | Example 12 |
| 6 | 0.15 | 7.2 | C | Comp. Example 2 |
| 7 | 0.11 | 2.8 | C | Comp. Example 2 |
| 8 | 0.58 | 3.7 | D | Comp. Example 3 |

| | | | | |
|---|---|---|---|---|
| *1: weight ratio of iodine compound included in liposome vesicles to liposome vesicular membrane lipid | | | | |

As apparent from Table 1, Sample 8 having liposome vesicular size larger than 0.5 µm resulted in a deteriorated cancer imaging level. On the contrary, Samples 1 to 5, which were prepared by dissolving phospholipid in supercritical carbon dioxide, exhibited superior cancer imaging levels to Samples 6 to 8 which were prepared by dissolution in organic solvents.

### Example 14

A mixture of 0.04 g of dipalmitoylphosphatidylcholine (DPPC), 1.2 mg of pluronic (F-88, available from ADEKA Co.) and 0.9 g of ethanol was charged into a stainless steel autoclave and the autoclave was internally heated to 60 °C, subsequently, 13 g of liquid carbon dioxide was added thereto. The pressure inside the autoclave was raised from 50 kg/cm² to 200 kg/cm² and the autoclave was internally stirred to allow the DPPC to be dissolved in supercritical carbon dioxide. To this supercritical carbon dioxide solution was continuously added with stirring a solution which contained 647 mg/ml of iohexol (having an iodine content of 300 mg/ml), 1.21 mg/ml of tromethamol and 0.1 mg/ml of edetate calcium disodium (EDTA Na₂-Ca) and which was also adjusted to a pH of ca. 7 with hydrochloric acid or sodium hydroxide. Thereafter, the inside of the autoclave was evacuated to discharge carbon dioxide, whereby a dispersion of liposomes containing iohexol was obtained. The obtained dispersion was heated to 60 °C and subjected to pressure filtration using a 0.1 µm cellulose type filter, produced by Advantech Co. The thus obtained contrast medium was designated as Sample 9. The average vesicular size of the liposome was 0.12 µm and the liposome was comprised substantially of unilamellar vesicles. The weight ratio of iodine compound included in vesicles to vesicle membrane lipid was 5.6.

Similarly to Example 14, liposome dispersions were prepared by subjecting it to pressure filtration using a 0.1 µm, 0.2 µm or 0.4 µm cellulose type filter and designated as Samples 10 to 15, except that the pressure was raised, the evacuation speed was controlled and the amount of the used phospholipid was varied.

Sample 16 was prepared similarly to Example 1, except that a mixture of 0.04 g of dipalmitoylphosphatidylcholine (DPPC), 0.01 g of cholesterol, 1.2 mg of pluronic (F-88, available from ADEKA Co.) and 0.9 g of ethanol was charged into a stainless steel autoclave. Sample 17 was prepared similarly to Sample 16, except that the pluronic F-88 was not used. Comparative Sample 18 was prepared similarly to Sample 9, except that the pluronic F-88 was not used. Each of Samples 9 to 18 was proved to be comprised of unilamellar vesicles.

Liposomes contained in the respective samples are shown in Table 2 with respect to liposome vesicular particle, weight ratio of iodine content of the iodine compound included in the liposome to lipid, and percentage of the iodine compound included in the liposome of the total amount of the iodine compound.

**Table 2**

| Sample No. | Average Vesicle Size (µm) | Included Iodine Compound | |
|---|---|---|---|
| | | Weight Ratio*¹ | Proportion (wt%)*² |
| 9 | 0.12 | 4.0 | 25 |
| 10 | 0.10 | 3.6 | 23 |
| 11 | 0.08 | 3.6 | 21 |
| 12 | 0.13 | 3.8 | 23 |
| 13 | 0.16 | 5.2 | 28 |
| 14 | 0.11 | 2.8 | 17 |
| 15 | 0.22 | 3.8 | 24 |
| 16 | 0.12 | 3.2 | 25 |
| 17 | 0.11 | 3.2 | 20 |
| 18 | 0.15 | 5.2 | 28 |

| | | | |
|---|---|---|---|
| *1: weight ratio of iodine compound included in liposome vesicles to liposome vesicle membrane lipid | | | |
| *2: weight percentage of iodine compound included in liposome vesicles, based on total iodine compound | | | |

### Example 15

Samples 9 to 18 were each added to a physiological salt solution and, after being sealed, heated to 42 °C and allowed to stand for 3 days. Thus aged samples and non-aged samples were observed using an optical microscope.

No minute particle was observed in any one of non-aged samples. In aged sample 18, liposome particles of several micrometers were observed, which were supposed to be formed by aggregation of liposome vesicles. Such liposome particles were also slightly observed in Sample 17. After being further aged for 4 days, such liposome particles were slightly observed in Samples 9 to 15 and no liposome particle was observed in Sample 16.

As can be seen from the foregoing results, the use of a contrast medium comprised of liposome containing a block copolymer of polyethylene oxide and polypropylene oxide or cholesterol led to enhanced stability.

### Example 16

Samples 9 to 18 were each diluted with an isotonic glucose solution to a concentration of 50 mg iodine/ml. When the thus obtained solution were each given to a rat by an intravenous injection, concentration to a liver was radiographically observed, specifically in Sample 17 and 18.

It was proved from the results that the use of the contrast medium containing liposome vesicles not using a block copolymer of polyethylene oxide and polypropylene oxide enabled selective imaging of a liver.

### Example 17

A cell suspension of VX2 calcinorma was hypodermically implanted into a rabbit. Two weeks after implantation, the contrast medium Samples 9 to 18 obtained in Example 14 were each given by an intravenous injection and then radiographically observed. In Samples 9, 10, 11, 12 14 and 16, a decrease of the imaging level or image contrast in the implant region of the rabbit was delayed, as compared to Sample 13, 15, 17 and 18.

### Example 18

A mixture of 0.032 g of dipalmitoylphosphatidylcholine (DPPC), 0.008 g of cholesterol, 1.2 mg of pluronic (F-88, available from ADEKA Co.) and 0.9 g of ethanol was charged into a stainless steel autoclave and the autoclave was internally heated to 60 °C, subsequently, 13 g of liquid carbon dioxide was added thereto. The pressure inside the autoclave was raised from 50 kg/cm² to 200 kg/cm² and the autoclave was internally stirred to allow the DPPC to be dissolved in supercritical carbon dioxide. To this supercritical carbon dioxide solution was continuously added with stirring a solution which contained 647 mg/ml of iohexol (having an iodine content of 300 mg/ml), 1.21 mg/ml of tromethamol and 0.1 mg/ml of edetate calcium disodium (EDTA Na₂-Ca) and which was also adjusted to a pH of ca. 7 with hydrochloric acid or sodium hydroxide. Thereafter, the inside of the autoclave was evacuated to discharge carbon dioxide, whereby a liposome dispersion containing iohexol was obtained. The obtained dispersion was heated to 60 °C and subjected to pressure filtration using a 0.1 µm cellulose type filter, produced by Advantech Co. The thus obtained contrast medium was designated as Sample 19. The average liposome vesicle size was 0.12 µm and the liposomes were substantially comprised of unilamellar vesicles. The weight ratio of the included iodine compound to the lipid of the liposome membrane was 5.6 and the iodine compound included in the liposome 23% by weight of the total iodine compound.

Similarly to Sample 19, liposome dispersions were prepared by having subjected to pressure filtration using a 0.1 µm, 0.2 µm and 0.4 µm cellulose type filters and designated as Samples 20 to 25, except that the pressure was raised, the evacuation speed was controlled and the amount of the used phospholipid was varied. Each of Samples 20 to 25 was proved to be comprised substantially of unilamellar vesicles. In Table 3, liposomes contained in the respective samples are shown with respect to liposome vesicular particle, weight ratio of iodine content of the iodine compound included in liposome vesicles to liposome vesicle membrane lipid, and percentage of the iodine compound included in the liposome of the total amount of the iodine compound.

Sample 26 was prepared similarly to sample 19, except that 0.024 g of dipalmitoylphosphatidylcholine (DPPC) and 0.006 g of cholesterol were used, and the pressure and evacuation speed were each adjusted.

Sample 27 was prepared similarly to sample 19, except that 0.048 g of dipalmitoylphosphatidylcholine (DPPC) and 0.012 g of cholesterol were used, and the pressure and the evacuation rate were each adjusted.

Samples 26 and 27 were each liposome of unilamellar vesicles. Liposomes contained in the respective samples are shown in Table 3, with respect to liposome vesicle, weight ratio of iodine content of the iodine compound included in the liposome vesicles to lipid, and percentage of the iodine compound included in liposome vesicles, based on the total amount of the iodine compound.

Samples 29 to 37 were prepared similarly to Samples 19 to 27, respectively, except that edetate disodium calcium (EDTA Na₂-Ca) was not used. Samples 29 and 37 were each liposome of unilamellar vesicles. Liposomes contained in the respective samples are shown in Table 3, with respect to liposome vesicle, weight ratio of iodine content of the iodine compound included in the liposome vesicles to lipid, and percentage of the iodine compound included in the liposome vesicles of the total amount of the iodine compound. As apparent therefrom, these samples exhibited nearly the same results as in the use of edetate disodium calcium.

Samples 39 to 47 were prepared similarly to Samples 19 to 27, respectively, except that tromethamol was not used. Samples 39 and 47 were each liposomes of unilamellar vesicles. Liposomes contained in the respective samples are shown in Table 3, with respect to liposome vesicle, weight ratio of iodine content of the iodine compound included in liposome vesicles to vesicle membrane lipid, and percentage of the iodine compound included in the liposome vesicles based on the total amount of the iodine compound. As apparent therefrom, these samples exhibited nearly the same results as in the use of tromethamol.

Comparative Samples 119 to 127, 129 to 137, and 139 to 147 were prepared similarly to Samples 19 to 27, 29 to 37, and 39 47, respectively, provided that liposome dispersion obtained in the respective samples was dialyzed using an isotonic salt solution so that the concentrations of the iodine compound and auxiliary additives contained in the aqueous medium of the liposome dispersion was lower than those included in the liposome. In Table 3, liposomes contained in the respective samples are shown with respect to average liposome vesicle size, weight ratio of iodine content of the iodine compound included in the liposome vesicles to membrane lipid, and percentage of the iodine compound included in the liposome vesicles based on the total amount of the iodine compound.

### Example 19

### Evaluation of Stability

Samples obtained in Example 18 were each added to a physiological salt solution and, after being sealed, they were heated to 42 °C and allowed to stand for 14 days. Thus aged samples and non-aged samples were observed using an optical microscope.

No minute particle was observed in any one of the non-aged samples. In some aged comparative samples, particles of several micrometers were observed, which were contemplated to be formed by aggregation of liposome vesicular particles. Aging stability was evaluated based on the following criteria:
5: no aggregated particle was observed,
4: a few aggregated particles were observed,
3: aggregated particles were readily apparent,
2: many aggregated particles were observed,
1: a large number of aggregated particles were observed.

### Example 20

As apparent from Table 3, it was proved that, when there is a difference in water-soluble component between inside and outside the liposome, the stability was lowered. Samples which were filtered using filters of 0.1 to 0.4 µm resulted in enhanced stability. On the contrary, when there is no difference in water-soluble component between inside and outside the liposome, enhanced stability was achieved in the presence of water-soluble amine type buffers or chelating agents.

**Table 3**

| Sample No. | Concentration Difference*¹ | Filtration*² | Average Vesicle Size (µm) | Included Iodine Compound | | Stability |
|---|---|---|---|---|---|---|
| | | | | Weight Ratio *³ | Proportion (wt%) *⁴ | |
| 19 | No | No | 0.12 | 3.7 | 23 | 4 |
| 20 | No | Yes | 0.07 | 3.4 | 21 | 5 |
| 21 | No | Yes | 0.095 | 3.2 | 20 | 5 |
| 22 | No | Yes | 0.12 | 3.8 | 24 | 5 |
| 23 | No | Yes | 0.15 | 3.7 | 23 | 5 |
| 24 | No | Yes | 0.18 | 4.0 | 25 | 5 |
| 25 | No | Yes | 0.21 | 3.9 | 24 | 5 |
| 26 | No | Yes | 0.12 | 6 | 28 | 5 |
| 27 | No | Yes | 0.12 | 2.5 | 23 | 5 |
| 29 | No | No | 0.12 | 3.7 | 23 | 4 |
| 30 | No | Yes | 0.07 | 3.4 | 21 | 4 |
| 31 | No | Yes | 0.095 | 3.2 | 20 | 4 |
| 32 | No | Yes | 0.12 | 3.8 | 24 | 4 |
| 33 | No | Yes | 0.15 | 3.7 | 23 | 4 |
| 34 | No | Yes | 0.18 | 4.0 | 25 | 4 |
| 35 | No | Yes | 0.21 | 3.9 | 24 | 4 |
| 36 | No | Yes | 0.12 | 6 | 28 | 4 |
| 37 | No | Yes | 0.12 | 2.5 | 23 | 4 |
| 39 | No | No | 0.12 | 3.7 | 23 | 4 |
| 40 | No | Yes | 0.07 | 3.4 | 21 | 4 |
| 41 | No | Yes | 0.095 | 3.2 | 20 | 4 |
| 42 | No | Yes | 0.12 | 3.8 | 24 | 4 |
| 43 | No | Yes | 0.15 | 3.7 | 23 | 4 |
| 44 | No | Yes | 0.18 | 4.0 | 25 | 4 |
| 45 | No | Yes | 0.21 | 3.9 | 24 | 4 |
| 46 | No | Yes | 0.12 | 6.0 | 28 | 4 |
| 47 | No | Yes | 0.12 | 2.5 | 23 | 4 |
| 119 | Yes | No | 0.12 | 3.7 | 23 | 2 |
| 120 | Yes | Yes | 0.07 | 3.4 | 21 | 3 |
| 121 | Yes | Yes | 0.095 | 3.2 | 20 | 3 |
| 122 | Yes | Yes | 0.12 | 3.8 | 24 | 3 |
| 123 | Yes | Yes | 0.15 | 3.7 | 23 | 3 |
| 124 | Yes | Yes | 0.18 | 4.0 | 25 | 3 |
| 125 | Yes | Yes | 0.21 | 3.9 | 24 | 2 |
| 126 | Yes | Yes | 0.12 | 6.0 | 28 | 2 |
| 127 | Yes | Yes | 0.12 | 2.5 | 23 | 3 |
| 129 | Yes | No | 0.12 | 3.7 | 23 | 2 |
| 130 | Yes | Yes | 0.07 | 3.4 | 21 | 3 |
| 131 | Yes | Yes | 0.095 | 3.2 | 20 | 3 |
| 132 | Yes | Yes | 0.12 | 3.8 | 24 | 3 |
| 133 | Yes | Yes | 0.15 | 3.7 | 23 | 3 |
| 134 | Yes | Yes | 0.18 | 4.0 | 25 | 2 |
| 135 | Yes | Yes | 0.21 | 3.9 | 24 | 2 |
| 136 | Yes | Yes | 0.12 | 6.0 | 28 | 2 |
| 137 | Yes | Yes | 0.12 | 2.5 | 23 | 3 |
| 139 | Yes | No | 0.12 | 3.7 | 23 | 4 |
| 140 | Yes | Yes | 0.07 | 3.4 | 21 | 2 |
| 141 | Yes | Yes | 0.095 | 3.2 | 20 | 2 |
| 142 | Yes | Yes | 0.12 | 3.8 | 24 | 2 |
| 143 | Yes | Yes | 0.15 | 3.7 | 23 | 2 |
| 144 | Yes | Yes | 0.18 | 4.0 | 25 | 2 |
| 145 | Yes | Yes | 0.21 | 3.9 | 24 | 2 |
| 146 | Yes | Yes | 0.12 | 6.0 | 28 | 2 |
| 147 | Yes | Yes | 0.12 | 2.5 | 23 | 2 |

| | | | | | | |
|---|---|---|---|---|---|---|
| *1: concentration difference in water-soluble component between inside and outside liposome | | | | | | |
| *2: filtration using filters of 0.1 µm, 0.2 µm and 0.4 µm pores | | | | | | |
| *3: weight ratio of iodine compound included in liposome vesicles to liposome vesicle membrane lipid | | | | | | |
| *4: weight percentage of iodine compound included in vesicles, based on total iodine compound | | | | | | |

## Claims

1. A radiographic contrast medium comprising liposomes including a water-soluble nonionic iodine compound, and the contrast medium is substantially free of a chlorine-containing solvent.

2. The contrast medium of claim 1, wherein the liposomes are prepared by a process of mixing a phospholipid with a supercritical carbon dioxide or a subcritical carbon dioxide and bringing a water-soluble nonionic iodine compound into contact with the phospholipid to form the liposome.

3. The contrast medium of claim 1, wherein the liposomes are comprised substantially of unilamellar vesicles.

4. The contrast medium of claim 1, wherein the iodine compound contains at least one 2,4,6-triiodopheny group.

5. The contrast medium of claim 1, wherein the liposomes have an average size of 0.05 to 0.5 µm.

6. The contrast medium of claim 5, wherein the average size is 0.05 to 0.2 µm.

7. The contrast medium of claim 6, wherein the average size is 0.11 to 0.13m.

8. The contrast medium of claim 1, wherein the liposomes are PEG-modified liposomes which are each comprises of a lipid membrane modified with a PEG having 10 to 3500 oxyethylene units in an amount of 0.1% to 30% by weight, based on lipid forming the liposomes.

9. The contrast medium of claim 1, wherein the liposomes are those which have been filtered with a filter having 0.1 to 0.4 µm pores.

10. The contrast medium of claim 1, wherein the liposomes include the iodine compound in a weight ratio of the iodine compound to liposome membrane lipid of 1 to 10.

11. The contrast medium of claim 10, wherein the weight ratio is 3 to 8.

12. The contrast medium of claim 11, wherein the weight ratio is 5 to 8.

13. The contrast medium of claim 1, wherein the iodine compound included in the liposomes accounts for 5% to 30% by weight of a total iodine compound amount of the contrast medium.

14. The contrast medium of claim 1, wherein the liposomes each comprise a lipid membrane and a water phase enclosed inside the lipid membrane, the lipid membrane comprises at least a compound selected from the group consisting of compounds containing a polyoxyalkylene oxide group and sterols and the water phase contains the iodine compound.

15. The contrast medium of claim 1, wherein the liposomes each comprise a lipid membrane and a water phase enclosed inside the lipid membrane, the lipid membrane comprises a phospholipid modified with a polyalkylene oxide and the water phase contains the iodine compound.

16. The contrast medium of claim 1, wherein the liposomes each comprise a lipid membrane and a water phase enclosed inside the lipid membrane, the lipid membrane comprises a block copolymer of polyethylene oxide and polypropylene oxide and the water phase contains the iodine compound.

17. The contrast medium of claim 1, wherein the liposomes which comprise a lipid membrane and a water phase enclosed inside the lipid membrane, are dispersed in an aqueous medium, both of the water phase and the aqueous medium contain the iodine compound and an additive and each of an iodine compound concentration and an additive concentration is substantially the same in both of the water phase and the aqueous medium.

18. The contrast medium of claim 17, wherein the additive is a water-soluble amine type buffering agents or a chelating agent.

19. The contrast medium of claim 18, wherein the amine type buffering agent is trometamol.

20. The contrast medium of claim 18, wherein the chelating agent is EDTA Na₂-Ca.

21. A method of preparing a radiographic contrast medium comprising the steps of:
mixing a phospholipid with a supercritical carbon dioxide or a subcritical carbon dioxide to form a mixture and
bringing a water-soluble nonionic iodine compound into contact with the phospholipid to form liposomes including the iodine compound.

22. The method of claim 21, wherein the method comprises the steps of:
mixing a phospholipid with a supercritical carbon dioxide or a subcritical carbon dioxide to form a mixture,
introducing an aqueous solution containing a nonionic iodine compound into the mixture and
discharging the carbon dioxide to form liposomes including the iodine compound.

23. The method of claim 21, wherein a pressure of 50 to 500 kg/cm² is applied to the carbon dioxide.

24. The method of claim 21, wherein a temperature of 25 to 200 °C is applied to the carbon dioxide.

25. The method of claim 22, wherein at least one selected from the group consisting of a phospholipid modified with a polyalkylene oxide, a compound containing a polyoxyalkylene group, a compound containing a polyethylene glycol group and a sterol is further mixed with the supercritical carbon dioxide or subcritical carbon dioxide, then, the aqueous solution containing a nonionic iodine compound is introduced into the mixture, and the carbon dioxide is discharged to form liposomes including the iodine compound; the method further comprises filtering the liposome with a filter having 0.1 to 0.4 µm pores.

26. The method of claim 22, wherein at least one selected from the group consisting of a phospholipid modified with a polyalkylene oxide and a sterol is further mixed with the supercritical carbon dioxide or subcritical carbon dioxide, then, the aqueous solution containing a nonionic iodine compound and an additive is introduced into the mixture and the carbon dioxide is discharged to form liposomes including the iodine compound and the additive; the method further comprises filtering the liposome with a filter having 0.1 to 0.4 µm pores to form liposomes which comprise a lipid membrane and a water phase included inside the lipid membrane, are dispersed in an aqueous medium, both of the water phase and the aqueous medium contain the iodine compound and the additive and each of an iodine compound concentration and an additive concentration is substantially the same in both of the water phase and the aqueous medium.
